# EUROPEAN PATENT APPLICATION

(11) **EP 4 825 314 A1**
(43) Date of publication of application: **21.10.2026**
(21) Application number: 25171103.2
(22) Date of filing: 16.04.2025
(51) Int. Cl.: A61B 5/11, A61B 5/346

(54) **METHODS AND SYSTEMS TO INTERPRET ECG CHANGES DUE TO BODY POSTURE**

(71) Applicant: Koninklijke Philips N.V., 5656 AE Eindhoven (NL)
(72) Inventor: STUT, Wilhelmus Johannes Joseph, 5656 AG Eindhoven (NL); EJUPI, Andreas, 5656 AG Eindhoven (NL); MAMPRIN, Marco, 5656 AG Eindhoven (NL)
(74) Representative: Philips Intellectual Property & Standards

(57) **Abstract**

A method (100) for ECG calibration, comprising: obtaining (120), while a body of an individual is positioned in a first posture, both: (i) first posture data from a posture component (280); and (ii) first ECG data from an ECG monitor (290); obtaining (130), while the body of the individual is positioned in a second posture different from the first posture, both: (i) second posture data from the posture component; and (ii) second ECG data from the ECG monitor; extracting (140), from the first ECG data, a plurality of first ECG waveforms; extracting (150), from the second ECG data, a plurality of second ECG waveforms; generating (160), from the extracted plurality of first ECG waveforms, a first ECG waveform baseline; generating (170), from the extracted plurality of second ECG waveforms, a second ECG waveform baseline; and reporting (180) one or both of the first ECG waveform baseline and the second ECG waveform baseline.

## Description

### FIELD OF THE INVENTION

The disclosed subject matter generally pertains to methods and systems for ECG calibration.

### BACKGROUND OF THE INVENTION

Electrocardiogram (ECG) is a non-invasive test that measures the electrical activity of the heart by placing electrodes on the chest and limbs. It is a vital diagnostic tool in cardiology, used to identify a wide range of cardiac conditions, including heart attack, arrhythmia, and heart failure. ECG can also be used to monitor the effectiveness of treatment and detect early signs of complications.

Mobile cardiac telemetry systems, for example, gather ECG data in ambulatory settings (e.g., in the home environment or on-the-go). For example, a mobile cardiac telemetry sensor can be placed in a patch on the chest and can communicate wirelessly with a dedicated smartphone which is connected (e.g., via a cellular network) to a clinical service center. Alternatively, the system may come without a smartphone, in which case the ECG data is uploaded when the sensor has been sent back by the patient to the hospital or vendor. In both cases, patient may use an app to record symptoms.

ECG signals can change depending on the posture of the individual being monitored. For example, studies have shown that the morphology of the ECG signal is different when the individual is in the upright posture versus a supine posture. When comparing supine and sitting postures, a marked change in ST-segment and T-wave morphology is observed. The ECG signal obtained from subjects in a seated posture showed a similar significant variation of the mean QRS-axis as compared to the supine posture, but differences in the magnitude of the changes differed with changes in body posture.

Although it is known that ECG signals can change depending on the posture of the individual, this information is not currently utilized by ECG systems as part of an ECG analysis.

### SUMMARY OF THE INVENTION

Thus, there is a continued need for ECG systems that utilize ECG calibration based on the posture of the subject's body. This results in significant improvements in analysis of the ECG trace for a subject.

Various embodiments and implementations are directed to methods and systems for ECG calibration using an ECG calibration system. The ECG calibration system obtains first ECG data and first posture data from an on-body posture component positioned on the body of the individual, while the individual is positioned in an upright posture. The system also obtains second ECG data and second posture data while the individual is positioned in a second posture different from the first posture, such as in a supine posture. The system extracts a plurality of ECG waveforms from the first ECG data and a plurality of waveforms from the second ECG data, and generates a first ECG waveform baseline and a second ECG waveform baseline, respectively. The system reports one or both of the first ECG waveform baseline and the second ECG waveform baseline.

According to an aspect, a method for ECG calibration is provided. The method includes: (i) obtaining, while a body of an individual is positioned in a first posture, both: (1) first posture data from an on-body posture component positioned on the body of the individual; and (2) first ECG data from an ECG monitor configured to obtain ECG data from the individual; (ii) obtaining, while the body of the individual is positioned in a second posture different from the first posture, both: (1) second posture data from the on-body posture component; and (2) second ECG data from the ECG monitor; (iii) extracting, from the first ECG data, a plurality of first ECG waveforms; (iv) extracting, from the second ECG data, a plurality of second ECG waveforms; (v) generating, from the extracted plurality of first ECG waveforms, a first ECG waveform baseline; (vi) generating, from the extracted plurality of second ECG waveforms, a second ECG waveform baseline; and (vii) reporting one or both of the first ECG waveform baseline and the second ECG waveform baseline.

In accordance with an embodiment, one of the first posture and the second posture is an upright posture, and the other of the first posture and the second posture is a supine posture.

In accordance with an embodiment, one of the first posture and the second posture is on the individual's side.

In accordance with an embodiment, the first posture data and the first ECG data are obtained after receiving a first notification from the individual that the individual is in the first posture, and further wherein the second posture data and the second ECG data are obtained after receiving a second notification from the individual that the individual is in the second posture. In accordance with an embodiment, the first notification and the second notification are received via an app of the ECG calibration system.

In accordance with an embodiment, the first posture data and the first ECG data are obtained after a posture algorithm of the ECG calibration system determines that the individual is in the first posture, and further wherein the second posture data and the second ECG data are obtained after the posture algorithm determines that the individual is in the second posture.

In accordance with an embodiment, the method further includes: extracting one or more individual characteristics from the generated first ECG waveform baseline; and extracting one or more individual characteristics from the generated second ECG waveform baseline.
In accordance with an embodiment, reporting further comprises some or all of the first ECG data and some or all of the second ECG data.

In accordance with an embodiment, reporting one or both of the first ECG waveform baseline and the second ECG waveform baseline comprises reporting a single aggregated signal for a plurality of heartbeats obtained from the first ECG data and/or a single aggregated signal for a plurality of heartbeats obtained from the second ECG data.

In accordance with another aspect, a system for ECG calibration is provided. The system includes: an on-body posture component positioned on a body of an individual; an ECG monitor configured to obtain ECG data from the individual; a processor configured to: (i) obtain, while the body of the individual is positioned in a first posture, both first posture data from the posture component and first ECG data from the ECG monitor; (ii) obtain, while the body of the individual is positioned in a second posture different from the first posture, both second posture data from the posture component and second ECG data from the ECG monitor; (iii) extract, from the first ECG data, a plurality of first ECG waveforms; (iv) extract, from the second ECG data, a plurality of second ECG waveforms; (v) generate, from the extracted plurality of first ECG waveforms, a first ECG waveform baseline; and (vi) generate, from the extracted plurality of second ECG waveforms, a second ECG waveform baseline; and a user interface configured to report one or both of the first ECG waveform baseline and the second ECG waveform baseline.

In accordance with an embodiment, the system further includes an app, wherein the first posture data and the first ECG data are obtained after receiving a first notification from the individual that the individual is in the first posture, and further wherein the second posture data and the second ECG data are obtained after receiving a second notification from the individual that the individual is in the second posture, and further wherein the first notification and the second notification are received or transmitted via the app.

In accordance with an embodiment, the system further includes a posture detection algorithm, wherein the first posture data and the first ECG data are obtained after the posture detection algorithm of the ECG calibration system determines that the individual is in the first posture, and further wherein the second posture data and the second ECG data are obtained after the posture algorithm determines that the individual is in the second posture.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1A is a flowchart of a method for ECG calibration, in accordance with an embodiment.
FIG. 1B is a flowchart of a method for ECG calibration, in accordance with an embodiment.
FIG. 2 is a schematic representation of an ECG calibration system, in accordance with an embodiment.
FIG. 3 is a schematic representation of an ECG calibration environment, in accordance with an embodiment.
FIG. 4 is a schematic representation of a reference matrix x, y, z acceleration values for postures of interest, in accordance with an embodiment.
FIG. 5A is a schematic representation of a report of ECG baseline, in accordance with an embodiment.
FIG. 5B is a schematic representation of a report of ECG baseline, in accordance with an embodiment.
FIG. 6 is an example of a report comprising ECG data and the posture of a subject's body in relation to the ECG data, in accordance with an embodiment.

### DETAILED DESCRIPTION OF EMBODIMENTS

The present disclosure describes various embodiments of a system and method configured for ECG calibration using an ECG calibration system. More generally, Applicant has recognized and appreciated that it would be beneficial to provide methods and systems for ECG calibration based on positioning of the subject's body. An ECG calibration system obtains first ECG data and first posture data from an on-body posture component positioned on the body of the individual, while the individual is positioned in an upright posture. The system also obtains second ECG data and second posture data while the individual is positioned in a second posture different from the first posture, such as in a supine posture. The system extracts a plurality of ECG waveforms from the first ECG data and a plurality of waveforms from the second ECG data, and generates a first ECG waveform baseline and a second ECG waveform baseline, respectively. The system reports one or both of the first ECG waveform baseline and the second ECG waveform baseline.

According to an embodiment, the systems and methods described or otherwise envisioned herein can, in some non-limiting embodiments, be implemented as a component of or an extension of ECG systems. For example, one application of the embodiments and implementations herein is to improve systems such as, e.g., the Philips^{®} ECG and/or IntelliSpace^{®} line of diagnostic and reporting tools (manufactured by Koninklijke Philips, N.V.), among many other products. However, the disclosure is not limited to these devices or systems, and thus the disclosure and embodiments disclosed herein can encompass any ECG method, device, or system.

According to an embodiment, state-of-the-art ECG devices contain or comprise an accelerometer that can be utilized to measure the posture of the patient, such as lying down (e.g., supine) or being in upright posture (sitting/standing). For example, if the sensor is placed in an upright posture, the y-axis measures close to 1 g (gravity). If the sensor is turned 90 degrees to the right or left, the y-axis measures close to 0 g. In general, the posture of the sensor in reference to gravity can be identified by roll, pitch and yaw angles. Showing posture information in the ECG report together with the ECG data and knowing how a patient's ECG changes with different body postures, will help improve diagnosis and treatment selection for the patient.

According to an embodiment, calibrating the ECG as described or otherwise envisioned herein provides numerous advantages over existing systems. For example, at the individual patient level, it is not known whether the upright ECG signal is different compared to the lying posture, and what the magnitude of the change is. With the systems and methods described herein, the effect of posture on the individual patient level is determined. As another example, when different patients are in an upright posture, the exact orientation of the accelerometers in their ECG monitors may be different. This is caused by multiple factors including: (1) with different versions of an ECG monitor, the accelerometer or other sensor may be placed slightly differently; (2) the ECG monitor may be used in different points on the body, and the actual posture or orientation of the monitor may be different in each of these different points; and (3) variation in body shape or composition. With the systems and methods described herein, the effect of posture on the patient's ECG signal can be determined.

Accordingly, provided herein are systems and methods to interpret ECG changes influenced by posture, utilizing an on-body calibration procedure, such as within a patient app. According to one possible embodiment, the method includes asking or directing the patient to sit or stand upright for a first period of time, and then to lie down for a second period of time. In both postures, the patient should not move. According to an embodiment, the patient app is used to indicate the start and end of the time period for each posture. The system automatically collects the (x,y,z) accelerometer values of these two reference postures, which can be later used to estimate and correct for the pitch, roll and yaw angles, and measures the ECG signal during these two intervals. The established ECG baselines in the standing and lying postures are shown in the ECG report and aim to improve the interpretation of ECG findings (e.g., for better relating changes to the ST segment).

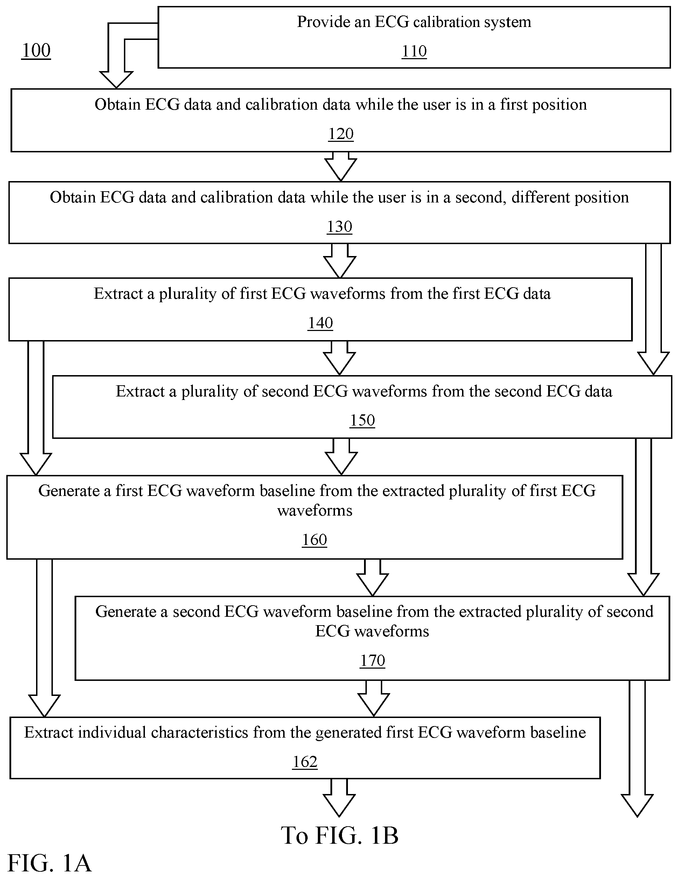

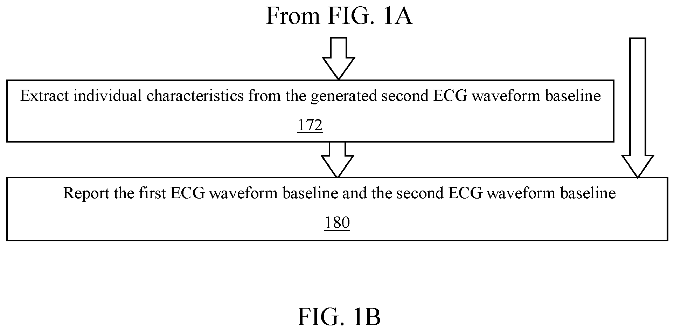

Referring to FIG. 1, in one embodiment, is a flowchart of a method 100 for ECG calibration using an ECG calibration system. The methods described in connection with the figures are provided as examples only, and shall be understood to not limit the scope of the disclosure. The ECG calibration system can be any of the systems described or otherwise envisioned herein. The ECG calibration system can be a single system or multiple different systems.

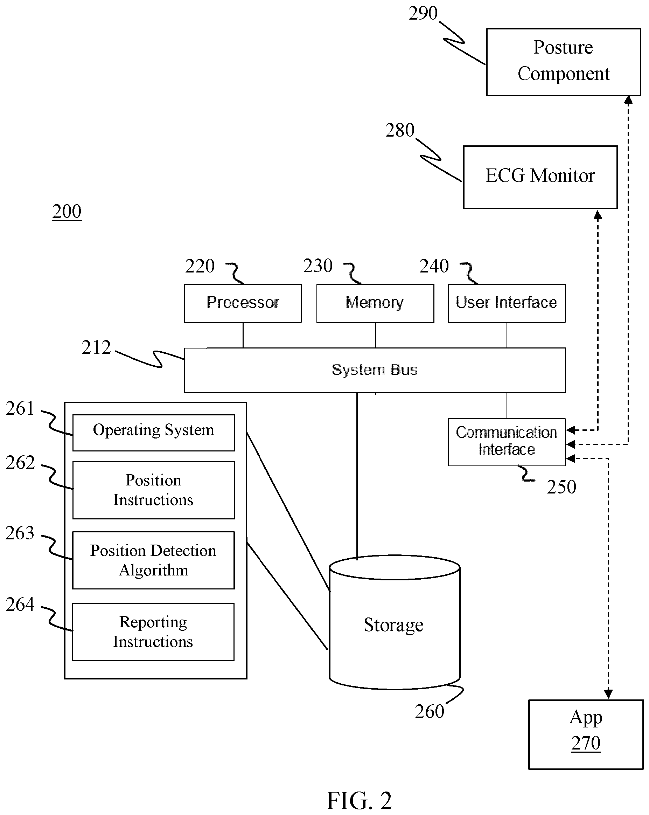

At step 110 of the method, an ECG calibration system 200 is provided. Referring to an embodiment of an ECG calibration system 200 as depicted in FIG. 2, for example, the system comprises one or more of a processor 220, memory 230, user interface 240, communication interface 250, and storage 260, interconnected via one or more system buses 212. It will be understood that FIG. 2 constitutes, in some respects, an abstraction and that the actual organization of the components of the system 200 may be different and more complex than illustrated. Additionally, ECG calibration system 200 can be any of the systems described or otherwise envisioned herein. Other elements and components of ECG calibration system 200 are disclosed and/or envisioned elsewhere herein.

According to an embodiment, the ECG calibration system 200 is or comprises or is in local and/or remote communication with a software application ("app") 270 which may be utilized as described or otherwise envisioned herein as part of the calibration system or method. The app 270 may be any app suitable to perform the tasks or procedures described or otherwise envisioned herein, and may be hosted on any computerized device including but not limited to a smartphone, desktop, laptop, tablet, wearable, smart speaker, smart mirror, and many other possible devices.

According to an embodiment, the ECG calibration system 200 is or comprises or is in local and/or remote communication with an ECG monitor 280. The ECG monitor 280 can be any ECG monitor, including but not limited to an ambulatory ECG monitor. An ambulatory ECG monitor is typically worn by a patient or subject for a period of time (hours, days, or weeks) as the patient or subject goes about their daily life, typically at home and work. Once the monitoring time period is complete, the patient or subject can provide the recorded ECG information to a healthcare professional. For example, the ECG monitor can upload the recorded ECG information (in real-time or in batches or all at once), or the patient or subject can return the ambulatory ECG monitor to the healthcare professional for download of the recorded ECG information. Notably, however, the methods and systems described or otherwise envisioned herein are not limited to ambulatory ECG monitors.

According to an embodiment, the ECG monitor 280-such as a Holter monitor or a patch-based monitor-continuously captures the electrical activity of the subject's heart over an extended period, often 24 hours or more. While the subject wears the monitor, which is typically lightweight and designed for everyday activities, the subject also has access to an application - such as a smartphone app - that optionally plays a crucial role in the cardiac evaluation. As described herein, the optionally app allows the subject to interact with the ECG system for purposes of calibration, and/or the app works behind the scenes without subject interaction in order to calibrate the system.

According to an embodiment, the ECG calibration system 200 is or comprises or is in local and/or remote communication with a posture component 290 that provides orientation or posture data. The posture component 290 can be any component or sensor capable of obtaining and providing orientation or posture data, including but not limited to an accelerometer. According to an embodiment, the posture component 290 is a component of the ECG monitor 280, although the component may optionally be a different element or component. For example, the posture component 290 may be a standalone component or may be an element of a posture or calibration device or component. Although shown as a separate component in FIG. 2, posture component 290 may be a component of the ECG monitor 280 or a component of another device or system of ECG calibration system 200.

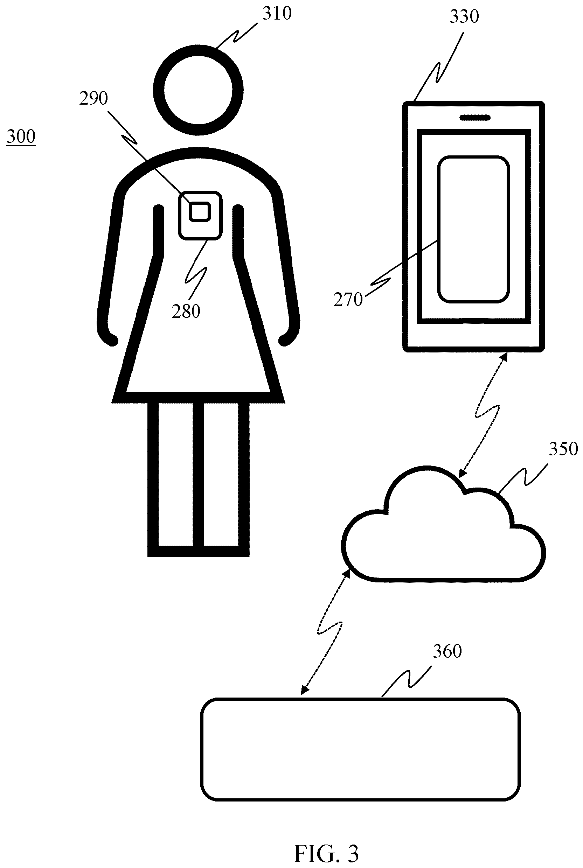

Referring to FIG. 3, in one embodiment, is an ECG monitoring environment 300. The ECG monitoring environment comprises a subject 310 that is being monitored using an ECG monitor 280, which may be an ambulatory ECG monitor or an in-house ECG monitor (e.g., an ECG monitor or system located within a healthcare setting). The environment further comprises a computerized device 330 with a software application ("app") 270, although the app may be hosted on any computerized device including but not limited to a smartphone, desktop, laptop, tablet, wearable, smart speaker, smart mirror, and many other possible devices.

According to an embodiment, for purposes of calibration, the app 270 can operate with or without the interaction of the subject 310 being monitored. As described herein, the system obtains posture data from a posture component 290 of the ECG monitor 280 or from another sensor, and records that posture data. The app may store this information, according to one possible embodiment. The computerized device 330 and/or app 270 can transmit the stored information via a wired and/or wireless communication system 350 to a remote component 360 of the ECG calibration system, such as a database or another application. This transmission of information can be in real-time, in batches, and/or all at once.

Returning to method 100 in FIG. 1, at step 120 of the method, the ECG calibration system 200 obtains, receives, or otherwise gets data while the body of the individual is positioned in a first posture. According to an embodiment, the first posture is an upright posture (although it can be another posture as described or otherwise envisioned herein). Upright can comprise several different postures or positions, including but not limited to seated upright and standing. According to an embodiment, the ECG calibration system obtains both (i) first posture data from the posture component 290 positioned on the body of the individual; and (ii) first ECG data from the ECG monitor 280 configured to obtain ECG data from the individual. According to an embodiment, the first posture data from the posture component 290 is posture or orientation data, such as from an accelerometer. Once obtained, the first posture data and the first ECG data may be utilized immediately or may be saved in local and/or remote memory for future use.

According to an embodiment, the first posture data and the first ECG data are obtained after receiving a first notification from the individual that the individual is in the upright posture. Some or all of the method can be mediated, influenced, directed, or otherwise instructed by directions or instructions from a calibration or other ECG app 270. Thus, before obtaining ECG data, the ECG app may perform a calibration as described or otherwise envisioned herein. For example, the subject or a healthcare professional may initiate the calibration by opening the ECG app or accessing a calibration interface of the ECG app. Once a calibration is initiated, the ECG app can provide instructions to the user (e.g., the subject or a healthcare professional), such as via a user interface or other communication mechanism. The ECG app can direct the user to apply one or more components of the ECG system, including but not limited to an ECG monitor 280 and/or the posture component 290 (which may be a single device or multiple devices). The app can provide advice or instructions for where and how to apply the one or more components.

Once applied, the components may detect that they have been applied or the user may inform the app that the components have been applied, such as by pressing a button, swiping, or otherwise interacting with the app via a user interface. The app can then instruct the user to assume a first posture, where the first posture is either upright (e.g., sitting upright or standing up), supine (e.g., on their back), on their side (e.g., left or right side), or another posture. The app can optionally provide instructions such as an image, video, text, or other instruction as to how to assume the first posture. Once the user has assumed that first posture, the user can then instruct the app that they are in the first posture, such as by pressing a button, swiping, or otherwise interacting with the app via a user interface. The app can instruct the user to stay in the first posture for a first period of time. The app can provide a countdown on a screen, and/or any other instructions relevant to the first posture and/or timing, as soon as the user indicates that they are in the first posture. The first period of time can be any amount of time necessary to obtain the relevant data. The first period of time can be seconds or minutes, for example. During the first period of time, the ECG system can obtain both (i) first posture data from the posture component 290 and (ii) first ECG data from the ECG monitor 280. The user can optionally inform the app when the first period of time is complete, or the app can automatically determine when the first period of time is complete using a timer or other timing mechanism.

According to another embodiment, the ECG calibration system comprises a posture algorithm 263. The posture algorithm is configured, programmed, or trained to determine when the wearer of the posture component 290 and/or ECG monitor 280 is in a desired posture, such as upright or supine. This can be determined, for example, without requiring the input or interaction of the user or wearer. The first posture data and the first ECG data can be obtained after the posture algorithm of the ECG calibration system determines that the individual is in the first posture (such as upright, supine, or on their side, among other options).

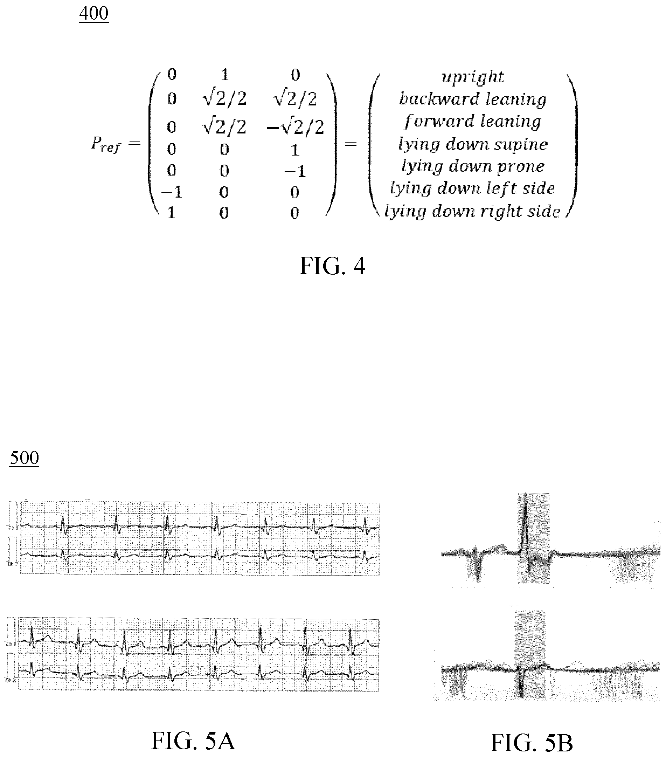

According to an embodiment, the posture algorithm can be capable of extracting ECG baselines at different postures throughout a day or night, therefore not requiring the user to perform a directed or instructed manual calibration. This can be achieved by the posture algorithm to automatically infer when the patient is in an upright or lying posture. One can then categorize similar ECG baselines per posture, yielding a set of ECG baselines per posture. According to an embodiment, the posture algorithm comprises a reference matrix of x, y, z acceleration values for the postures of interest and matches a detected posture with one of the references in the matrix. Referring to FIG. 4, in one embodiment, is a reference matrix 400 of x, y, z acceleration values for the postures of interest. According to an embodiment, the posture algorithm may alternatively operate according to another mechanism.

Returning to method 100 in FIG. 1, at step 130 of the method, the ECG calibration system 200 obtains, receives, or otherwise gets data while the body of the individual is positioned in a second posture. The second posture is different from the first posture. According to an embodiment, the second posture is a supine posture (although it can be another posture as described or otherwise envisioned herein). Supine can include the subject lying on their back. According to an embodiment, the ECG calibration system obtains both (i) second posture data from the posture component 290 positioned on the body of the individual; and (ii) second ECG data from the ECG monitor 280 configured to obtain ECG data from the individual. According to an embodiment, the second posture data from the posture component 290 is posture or orientation data, such as from an accelerometer. Once obtained, the second posture data and the second ECG data may be utilized immediately or may be saved in local and/or remote memory for future use.

According to an embodiment, the second posture data and the second ECG data are obtained after receiving a second notification from the individual that the individual is in the supine posture. Some or all of the method can be mediated, influenced, directed, or otherwise instructed by directions or instructions from the calibration or other ECG app 270. The app can instruct the user to assume the second posture, where the first posture is either upright (e.g., sitting upright or standing up), supine (e.g., on their back), on their side (e.g., left or right side), or another posture. The app can optionally provide instructions such as an image, video, text, or other instruction as to how to assume the second posture. Once the user has assumed that second posture, the user can then instruct the app that they are in the second posture, such as by pressing a button, swiping, or otherwise interacting with the app via a user interface. The app can instruct the user to stay in the second posture for a second period of time, which can be the same as or different from the first period of time. The app can provide a countdown on a screen, and/or any other instructions relevant to the second posture and/or timing, as soon as the user indicates that they are in the second posture. The second period of time can be any amount of time necessary to obtain the relevant data. The second period of time can be seconds or minutes, for example. During the second period of time, the ECG system can obtain both (i) second posture data from the posture component290 and (ii) second ECG data from the ECG monitor 280. The user can optionally inform the app when the second period of time is complete, or the app can automatically determine when the second period of time is complete using a timer or other timing mechanism.

According to another embodiment, the ECG calibration system comprises a posture algorithm 263. The posture algorithm is configured, programmed, or trained to determine when the wearer of the posture component 290 and/or ECG monitor 280 is in a desired position, such as upright or supine. This can be determined, for example, without requiring the input or interaction of the user or wearer. The second posture data and the second ECG data can be obtained after the posture algorithm of the ECG calibration system determines that the individual is in the second posture (such as upright, supine, or on their side, among other options).

According to an embodiment therefore, the user can inform the app about the start and stop of the first and second intervals in different ways, such as pressing a button (e.g., "start upright") on the screen, or using voice recognition (e.g., saying "end lying"). The app can communicate the four timestamps (corresponding to start first posture, end first posture, start second posture, end second posture) to the cloud/backend via the smartphone's data network connection. If the system is connected to the cloud/backend (e.g., via a dedicated smartphone or other component), the ECG baselines and accelerometer data in the first and second postures are sent to the cloud/backend when the last step has been executed. If the system is not connected to the cloud/backend, the ECG baselines and accelerometer data in the first and second postures are uploaded to the cloud/backend when the patient has returned the monitor to the hospital or vendor, or when the system otherwise connects to the cloud/backend.

Notably, steps 120 and 130 can be reversed, in that first posture data and first ECG data may first be obtained while the body of the individual is in the supine posture followed by obtaining second posture data and second ECG data while the body of the individual is in the upright posture. Alternatively, one of the first posture and the second posture can be the user lying on their side, among other postures.

At step 140 of the method, the system extracts a plurality of first ECG waveforms from the first ECG data. The plurality of first ECG waveforms represents ECG waveforms that are or will be obtained while the individual is in the first posture (which is upright, supine, or on the side, among other possible postures). The plurality of first ECG waveforms can be extracted from the first ECG data using any method for identifying and/or extracting ECG waveforms from an ECG trace. The plurality of first ECG waveforms can be extracted from the first ECG data at any point, including while the first ECG data is being obtained, or after the first ECG data has been obtained. Once extracted, the plurality of first ECG waveforms can be used immediately or can be stored in local and/or remote memory for downstream use.

Similarly, at step 150 of the method, the system extracts a plurality of second ECG waveforms from the second ECG data. The plurality of second ECG waveforms represents ECG waveforms that are or will be obtained while the individual is in the second posture (which is upright, supine, or on the side, among other possible postures), where the second posture is different from the first posture. The plurality of second ECG waveforms can be extracted from the second ECG data using any method for identifying and/or extracting ECG waveforms from an ECG trace. The plurality of second ECG waveforms can be extracted from the second ECG data at any point, including while the second ECG data is being obtained, or after the second ECG data has been obtained. Once extracted, the plurality of second ECG waveforms can be used immediately or can be stored in local and/or remote memory for downstream use.

At step 160 of the method, the ECG system generates a first ECG waveform baseline from the extracted plurality of first ECG waveforms. The first ECG waveform baseline can be formed using any method for forming a baseline, including the methods described or otherwise envisioned herein. Once generated, the first ECG waveform baseline can be used immediately or can be stored in local and/or remote memory for downstream use.

At optional step 162 of the method, the ECG system uses the generated first ECG waveform baseline to extract one or more individual characteristics. The one or more individual characteristics can be any parameters or characteristics that can be identified or otherwise extracted from an ECG waveform baseline. For example, the one or more individual characteristics can include - but are not limited to - characteristics such as heart rate among others.

At step 170 of the method, the ECG system generates a second ECG waveform baseline from the extracted plurality of second ECG waveforms. The second ECG waveform baseline can be formed using any method for forming a baseline, including the methods described or otherwise envisioned herein. Once generated, the second ECG waveform baseline can be used immediately or can be stored in local and/or remote memory for downstream use.

At optional step 172 of the method, the ECG system uses the generated second ECG waveform baseline to extract one or more individual characteristics. The one or more individual characteristics can be any parameters or characteristics that can be identified or otherwise extracted from an ECG waveform baseline. For example, the one or more individual characteristics can include - but are not limited to - characteristics such as heart rate, among others.

Therefore, according to an embodiment, once the appropriate postures are identified (using any of the methods described or otherwise envisioned herein), it is possible to extract a large number of ECG waveforms for the first and second postures, and to create ECG baselines. One can then derive characteristics of each ECG baseline such as heart rate range (e.g., 70-80 bpm, 80-90 bpm, etc.), among many other possible characteristics. For both the first and second postures, the ECG baselines can then be grouped based on their average heart rate, or other characteristics.

For each baseline group, a display or other visualization can be generated, as described herein. Thus, at step 180 of the method, the ECG system reports one or both of the first ECG waveform baseline and the second ECG waveform baseline. A waveform baseline can be communicated in a wide variety of different ways. For example, according to an embodiment, reporting one or both of the first ECG waveform baseline and the second ECG waveform baseline comprises reporting a single aggregated signal for a plurality of heartbeats obtained from the first ECG data and/or a single aggregated signal for a plurality of heartbeats obtained from the second ECG data. According to another embodiment, the ECG report can show a corresponding ECG baseline close to or otherwise with an ECG strip, depending on the patient posture(s) and heart rate range(s) during the strip.

According to an embodiment, an ECG baseline can be reported in numerous different ways. For example, referring to FIGS. 5A and 5B, in one embodiment, are methods for reporting an ECG baseline. In FIG. 5A, the ECG baseline for a first posture (upright) is shown in the top panel, and the ECG baseline for a second posture (supine) is shown in the bottom panel. The ECG baselines are shown for the entire strip (of x and y seconds, respectively). In FIG. 5B, the ECG baseline for a first posture (upright) is shown in the top panel, and the ECG baseline for a second posture (supine) is shown in the bottom panel. The ECG baselines are shown as an aggregated signal of one heartbeat.

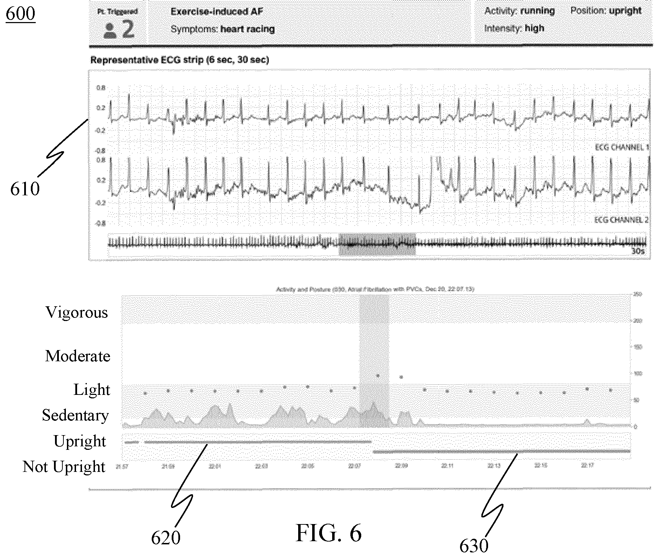

Referring to FIG. 6, in one embodiment, the posture of the subject's body in relation to obtained ECG data can is shown in a report. The report includes an ECG trace 610, and includes posture data (upright 620 and not upright 630) relative to the trace. The report also includes information about the activity level of the user relative to the trace and the posture data.

Referring to TABLE 1, in one embodiment, is an example workflow or pseudocode for a method for generating and reporting baselines, using heart rate as a derived characteristic.

**TABLE 1. Example Workflow or Pseudocode**

| | |
|---|---|
| 1. Load ECG and Accelerometer Data | Load the ECG signals and corresponding accelerometer data for the first 24 hours |
| 2. Detect Posture | Use the accelerometer data to classify when the patient was either upright or lying down |
| 3. Divide ECG Data into Baselines | Divide the ECG data into baselines. Baselines can be defined in different ways. For example: they can have a certain duration (e.g., 10 seconds), or span a certain number of heart beats (e.g., 1 or 4) |
| 4. Compute Average Heart Rate | For each ECG baseline, compute the average heart rate |
| 5. Categorize ECG Baselines | Based on the posture classification, separate the ECG baselines into two lists: upright and lying down |
| 6. Group ECG Baselines | Group the upright ECG baselines by average heart rate, activity levels, time of the day or other characteristics. |
| | Group the lying ECG baselines by average heart rate, activity levels, time of the day or other characteristics |

According to an embodiment, during an ECG study, patients typically use multiple patches since each patch only lasts for a limited number of days (e.g., 5 days). These days may not be calendar days, but may start at 10 AM and end at 2 PM, as one example. Many other start and stop times are possible. Depending on the connectivity of the monitor, ECG reports may be generated on a daily basis or at the end of the service.

According to an embodiment of a system with a connected monitor and optional daily reports, the system can construct ECG baselines during a first 24 hours after applying a new patch, although of course other time periods are possible. In this embodiment, the construction of ECG baselines could be performed immediately during the first 24 hours, which is sufficient to provide about 24h*60m*60s = 8640 segments of 10s. By doing this in the first 24 hours, the baseline ECG views are available for the daily reports that come with connected devices.

According to an embodiment of a system with a non-connected monitor and a report provided at the end of the service or when a connection is made, the system can construct ECG baselines per patch-wearing period. For a non-connected monitor the processing can be done when the data are ultimately uploaded. However, because in this case a new patch is potentially placed multiple times, every time in a slightly different location or posture, the extraction of the ECG waveforms and the creation of the ECG baselines should be performed per patch wearing period (of circa 5*24=120 hours).

Referring again to FIG. 2 is a schematic representation of an ECG calibration system 200. System 200 may be any of the devices or systems described or otherwise envisioned herein, and may comprise any of the components described or otherwise envisioned herein. It will be understood that FIG. 2 constitutes, in some respects, an abstraction and that the actual organization of the components of system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, storage 260 of system 200 may store one or more algorithms, modules, and/or instructions to carry out one or more functions or steps of the methods described or otherwise envisioned herein. For example, storage 260 may comprise, among other instructions or data, posture instructions 262, posture detection algorithm 263, and/or reporting instructions 264.

According to an embodiment, posture instructions 262 direct the system to provide posture information or instructions or directions to the user, such as via app 270, although the instructions may be provided using any other mechanism for communicating information to a user. The posture information or instructions can be, for example, instructions for the user to assume a first posture for a first period of time, and to assume a second posture for a second period of time. The posture information or instructions can include a visual, audible, and/or text-based instruction, and can include a timer or other indication of the respective time period. The posture information or instructions can further be instructions for the user to assume a third posture for a third period of time, and so on. The posture information or instructions can be any of the directions or instructions described or otherwise envisioned herein.

According to an embodiment, posture detection algorithm 263 is configured, programmed, or trained to determine when the wearer of the posture component 290 and/or ECG monitor 280 is in a desired posture, such as upright or supine. This can be determined, for example, without requiring the input or interaction of the user or wearer. The posture data and the ECG data can be obtained after the posture algorithm of the ECG calibration system determines that the individual is in the proper posture (such as upright, supine, or on their side, among other options). According to an embodiment, the posture algorithm is capable of extracting ECG baselines at different postures throughout a day or night, therefore not requiring the user to perform a directed or instructed manual calibration. This can be achieved by the posture algorithm to automatically infer when the patient is in an upright or lying posture. One can then categorize similar ECG baselines per posture, yielding a set of ECG baselines per posture. According to an embodiment, the posture algorithm comprises a reference matrix of x, y, z acceleration values for the postures of interest and matches a detected posture with one of the references in the matrix. According to an embodiment, the posture algorithm may alternatively operate according to another mechanism.

According to an embodiment, reporting instructions 264 direct the system to provide a report of any of the information generated or obtained as described or otherwise envisioned herein. For example, the report can include one or both of the first ECG waveform baseline and the second ECG waveform baseline. A waveform baseline can be communicated in a wide variety of different ways. For example, according to an embodiment, reporting one or both of the first ECG waveform baseline and the second ECG waveform baseline comprises reporting a single aggregated signal for a plurality of heartbeats obtained from the first ECG data and/or a single aggregated signal for a plurality of heartbeats obtained from the second ECG data. According to another embodiment, the ECG report can show a corresponding ECG baseline close to or otherwise with an ECG strip, depending on the patient posture(s) and heart rate range(s) during the strip.

Thus, the provided output can be any of the information as described or otherwise envisioned herein. The system may provide the information to a user via any mechanism, including but not limited to a visual display. The information may be communicated by wired and/or wireless communication to another device. For example, the system may communicate the information to a monitor, screen, mobile phone, computer, laptop, wearable device, and/or any other device configured to allow display and/or other communication of the information.

It should be appreciated that all combinations of the foregoing concepts and additional concepts discussed in greater detail herein (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for ECG calibration using an ECG calibration system (200), comprising:
obtaining (120), while a body of an individual is positioned in a first posture, both: (i) first posture data from an on-body posture component (280) positioned on the body of the individual; and (ii) first ECG data from an ECG monitor (290) configured to obtain ECG data from the individual;
obtaining (130), while the body of the individual is positioned in a second posture different from the first posture, both: (i) second posture data from the on-body posture component; and (ii) second ECG data from the ECG monitor;
extracting (140), from the first ECG data, a plurality of first ECG waveforms;
extracting (150), from the second ECG data, a plurality of second ECG waveforms;
generating (160), from the extracted plurality of first ECG waveforms, a first ECG waveform baseline;
generating (170), from the extracted plurality of second ECG waveforms, a second ECG waveform baseline; and
reporting (180) one or both of the first ECG waveform baseline and the second ECG waveform baseline.

2. The method of claim 1, wherein one of the first posture and the second posture is an upright posture, and the other of the first posture and the second posture is a supine posture.

3. The method of claim 1, wherein one of the first posture and the second posture is on the individual's side.

4. The method of claim 1, wherein the first posture data and the first ECG data are obtained after receiving a first notification from the individual that the individual is in the first posture, and further wherein the second posture data and the second ECG data are obtained after receiving a second notification from the individual that the individual is in the second posture.

5. The method of claim 4, wherein the first notification and the second notification are received via an app (270) of the ECG calibration system.

6. The method of claim 1, wherein the first posture data and the first ECG data are obtained after a posture algorithm (263) of the ECG calibration system determines that the individual is in the first posture, and further wherein the second posture data and the second ECG data are obtained after the posture algorithm determines that the individual is in the second posture.

7. The method of claim 1, further comprising the steps of:
extracting (162) one or more individual characteristics from the generated first ECG waveform baseline; and
extracting (172) one or more individual characteristics from the generated second ECG waveform baseline.

8. The method of claim 1, wherein reporting further comprises some or all of the first ECG data and some or all of the second ECG data.

9. The method of claim 1, wherein reporting one or both of the first ECG waveform baseline and the second ECG waveform baseline comprises reporting a single aggregated signal for a plurality of heartbeats obtained from the first ECG data and/or a single aggregated signal for a plurality of heartbeats obtained from the second ECG data.

10. A system (200) for ECG calibration, comprising:
an on-body posture component (280) positioned on a body of an individual;
an ECG monitor (290) configured to obtain ECG data from the individual;
a processor (220) configured to: (i) obtain, while the body of the individual is positioned in a first posture, both first posture data from the posture component and first ECG data from the ECG monitor; (ii) obtain, while the body of the individual is positioned in a second posture different from the first posture, both second posture data from the posture component and second ECG data from the ECG monitor; (iii) extract, from the first ECG data, a plurality of first ECG waveforms; (iv) extract, from the second ECG data, a plurality of second ECG waveforms; (v) generate, from the extracted plurality of first ECG waveforms, a first ECG waveform baseline; and (vi) generate, from the extracted plurality of second ECG waveforms, a second ECG waveform baseline; and
a user interface (240) configured to report one or both of the first ECG waveform baseline and the second ECG waveform baseline.

11. The system of claim 10, wherein one of the first posture and the second posture is an upright posture, and the other of the first posture and the second posture is a supine posture.

12. The system of claim 10, wherein one of the first posture and the second posture is on the individual's side.

13. The system of claim 10, further comprising:
an app (270), wherein the first posture data and the first ECG data are obtained after receiving a first notification from the individual that the individual is in the first posture, and further wherein the second posture data and the second ECG data are obtained after receiving a second notification from the individual that the individual is in the second posture, and further wherein the first notification and the second notification are received via the app.

14. The system of claim 10, wherein the first posture data and the first ECG data are obtained after a posture algorithm (263) of the ECG calibration system determines that the individual is in the first posture, and further wherein the second posture data and the second ECG data are obtained after the posture algorithm determines that the individual is in the second posture.

15. The system of claim 10, wherein reporting one or both of the first ECG waveform baseline and the second ECG waveform baseline comprises reporting a single aggregated signal for a plurality of heartbeats obtained from the first ECG data and/or a single aggregated signal for a plurality of heartbeats obtained from the second ECG data.
